# EUROPEAN PATENT APPLICATION

(11) **EP 1 914 552 A1**
(43) Date of publication of application: **23.04.2008**
(21) Application number: 06122704.7
(22) Date of filing: 20.10.2006
(51) Int. Cl.: G01N 33/68

(54) **Method for identifying women with an increased risk of pre-eclampsia**

(71) Applicant: THE UNIVERSITY OF NEWCASTLE RESEARCH ASSOCIATES LIMITED, Callaghan, NSW 2308 (AU)
(72) Inventor: Smith, Roger The University of Newcastle, Newcastle New South Wales 2310 (AU)
(74) Representative: Habets, Winand

(57) **Abstract**

The present invention relates to methods for determining or aiding in the determination that a pregnant woman is at risk of developing pre-eclampsia. In certain embodiments, the invention relates to methods for determining or aiding in the determination that a pregnant woman has pre-eclampsia, in further embodiments, the invention relates to methods of screening or pre-screening pregnant women to identify those pregnant women with a low risk of developing hypertensive disorders, which reduces the need for additional testing throughout the pregnancy.

## Description

### Field of the invention

This invention relates to methods for the detection of pre-eclampsia. In particular, this invention is directed to determining an early indication of increased risk of pre-eclampsia by detecting an abnormal level of a number of biomarkers in particular in the serum, plasma or blood of pregnant women.

### Background of the invention

Pre-eclampsia is a hypertensive disorder that complicates up to 6-8% of pregnancies and remains the leading cause of maternal and perinatal morbidity and mortality (Roberts JM, Cooper DW. Pathogenesis and genetics of pre-eclampsia. Lancet. 2001; 357(9249):53-56. and MacKay AP, Berg CJ, Atrash HK. Pregnancy- related mortality from pre-eclampsia and eclampsia. Obstet Gynecol. 2001;97:533-38). Yet, despite extensive research efforts, the etiology of this multi-systematic disorder remains incompletely understood. Vascular endothelial activation followed by vasospasm appears to be the central feature in the pathogenesis of pre-eclampsia.

Theories of its cause include abnormal implantation and development of the placenta, oxidative stress, impaired endothelial prostanoid and nitric oxide homeostasis, genetic polymorphisms, abnormal circulating autoantibodies and an abnormal maternal systematic inflammatory response (Buhimschi IA, Saade GR, Chwalisz K, Garfield RE. The nitric oxide pathway in pre-eclampsia: pathophysiological implications. Hum Reprod Update, 1998;4:25-42, Ward K, Hata A, Jeunemaitre X, Helin C, Nelson L, Namikawa C, Farrington PF, et al.. A molecular variant of angiotensinogen associated with pre-eclampsia. Nat Genet 1993;4:59, Wallukat G, Homuth V, Fischer T, Horstkamp B, Jupner A, Baur E, Nissen E, Vetter K, Dudenhausen JW, Haller H, Luft FC. Patients with pre-eclampsia develop agonistic antibodies against the angiotensin AT 1 receptor. J Clin Invest 1999; 103:945-952., Fass MM, Schinling GA, Bailer JFW, Visscher CA, Bakker WW. A new animal model for human pre-eclampsia: ultra-low dose of endotoxin infusion in pregnant rats. Am J Obstet Gynecol 1994; 171: 158-64., Roberts JM, Redman CW. Pre-eclampsia: more than pregnancy induced hypertension. Lancet 1993; 341: 1447-51).

More recently, there has been increased focus on the effects of variations in the expression levels of modulators of angiogenesis, which cause symptoms of pre-eclampsia, including hypertension, proteinuria, endothelial cell activation and increased platelet aggregation ( Maynard SE, Min JY, Merchan J, Lim KH, Li J, Mondal S et al. Excess placental soluble fms-like tyrosine kinase 1 (sFltl) may contribute to endothelial dysfunction, hypertension, and proteinuria in pre-eclampsia. J Clin Invest 2003; 111 : 649-58., Volhard F: Die doppelseitigen haematogenen Nierenerkrankungen Beril, Springer. 1918., Buhimschi IA, Saade GR, Chwalisz K, Garfield RE. The nitric oxide pathway in pre-eclampsia: pathophysiological implications. Hum Reprod Update. 1998;4:25-42. Takacs P, Kauma SW, Sholley MM, Walsh SW, Dinsmoor MJ, Green K. Increased circulating lipid peroxides in severe preeclampsia activate NF-kappaB and upregulate ICAM-I in vascular endothelial cells. FASEB J. 2001 ;15:279-81., Lockwood CJ, Peters JH. Increased plasma levels of EDI+ cellular fibronectin precede the clinical signs of pre-eclampsia. Am J Obstet Gynecol. 1990;162:358- 62.)

Specifically, recent studies have reported that maternal serum concentrations of vascular endothelial growth factor (VEGF), placental growth factor (PIGF) and soluble fins- like tyrosine kinase-1 (sFlt-1) are altered in patients with clinical pre-eclampsia (Levine RJ, Maynard SE, Qian C, Lim KH, England LJ, Yu KF, et al. Circulating angiogenic factors and the risk of pre-eclampsia. N Engl J Med 2004:12;350:672-83., Maynard SE, Min JY, Merchan J, Lim KH, Li J, Mondal S et al. Excess placental soluble fins-like tyrosine kinase 1 (sFltl) may contribute to endothelial dysfunction, hypertension, and proteinuria in pre-eclampsia. J Clin Invest 2003; 111 :649-58., Levine RJ, Maynard SE, Qian C, Lim KH, England LJ, Yu KF, et al. Circulating angiogenic factors and the risk of pre-eclampsia. N Engl J Med 2004: 12; 350: 672-83.)

Neurologic manifestations are common in severe pre-eclampsia, and include seizures or coma (eclampsia), stroke, hypertensive encephalopathy, headaches, and visual aberrations. Despite intensive research efforts, the etiology of pre-eclampsia and its neurologic manifestations remains unknown.

Pre-eclampsia can vary in severity from mild to life threatening. A mild form of pre-eclampsia can be treated with bed rest and frequent monitoring. For moderate to severe cases, hospitalization is recommended and blood pressure medication or anticonvulsant medications to prevent seizures are prescribed.

The symptoms of pre-eclampsia typically appear after the 20th week of pregnancy and are usually detected by routine monitoring of the woman's blood pressure and urine. However, these monitoring methods are ineffective for diagnosis of the syndrome at an early stage, which could reduce the risk to the subject or developing fetus, if an effective treatment were available.

Currently, there are no known cures for pre-eclampsia. The only definitive treatment for pre-eclampsia is delivery of the fetus and placenta. Hospitalization, strict bed rest, magnesium sulfate administration to prevent convulsions, and prompt delivery remain as the current standard of therapy for preeclampsia.

Currently, there is no single test to predict or diagnose pre-eclampsia or to predict the severity of the condition that will develop in a particular patient. Early symptoms include persistent headaches, blurred vision or sensitivity to light and abdominal pain. However, a diagnosis of pre-eclampsia is not typically made until increased blood pressure and protein in the urine (proteinuria) are revealed, typically in routine physician tests following the 20th week of pregnancy. Severe effects of preeclampsia, including seizures, cerebral hemorrhage, disseminated intravascular coagulation and renal failure, may appear very shortly following such diagnosis. These methods are imprecise and provide little insight into the likelihood of the most severe symptoms developing. Moreover, the current diagnostics require physician oversight and invasive methodologies, further delaying and complicating early and immediate assessment.

Recently, a number of diagnostic methods for determing preeclampsia have been described. WO 90/07122 describes the diagnosis of preeclampsia using an assay to measure a mitogenic factor in blood. This assay incorporates the culturing of animal fibroblasts or smooth muscle cells and is therefore a time consuming and elaborate method.

US patent 5,849,474 discloses a method for the diagnosis of preeclampsia wherein the levels of a hemoglobin variant and a red blood cell glycolytic enzyme are measured. It remains unclear whether this method may be used to identify women at risk for pre-eclampsia.

WO 02/04678 provides methods for diagnosing pre-eclampsia wherein the levels of syncytin mRNA expression or polypeptide are measured.

WO 2005/093413 provides a method for the diagnosis of preeclampsia wherein the level of free hemoglobin is measured in a cerebrospinal fluid sample.

US patent application 2005/0255114 describes a proteomics approach towards the identification of patients at risk of pre-eclampsia. It provides a number of 852 candidate proteins that may exhibit either elevated or decreased levels of expression in patients with pre-eclampsia.

WO 2005/078128 is directed towards a method for determining whether a subject is at increased risk for pre-eclampsia by detecting the presence or absence of a variant genotype of the DDAH gene in a biological sample.

US patent application 2005/0074746 relates to a method for determining if a pregnant womam is at risk of developing pre-eclampsia wherein human trophoblast cells are cultured in the presence of serum or plasma of a pregnant woman.

WO 2006/069373 teaches a method of diagnosing a pregnant woman as having or being susceptible to developing a hypertensive disorder. This method involves the measurement of levels of soluble fms-like tyrosine kinase 1 in a urine sample.

Despite of all the above efforts, there is an urgent need for an early and accurate method for the detection and diagnosis of pre-eclampsia and associated proteinuric hypertensive disorders.

### Summary of the invention

The present invention relates to methods for determining or aiding in the determination that a pregnant woman is at risk of developing pre-eclampsia or other hypertensive disorder(s). In certain embodiments, the invention relates to methods for determining or aiding in the determination that a pregnant woman has preeclampsia, in further embodiments, the invention relates to methods of screening or pre-screening pregnant women to identify those pregnant women with a low risk of developing hypertensive disorders, which reduces the need for additional testing throughout the pregnancy.

In more detail, the invention relates to a method for identifying women with an increased risk of pre-eclampsia comprising the steps of obtaining a biological sample from a pregnant patient after week 12 and prior to week 37 of pregnancy, in the biological sample, determining the level of at least one biomarker selected from the group consisting of alpha-1B-glycoprotein, antithrombin III and transthyretin, comparing the level of the at least one biomarker with a level characteristic of normal pregnancies, wherein an abnormal level of said at least one biomarker is indicative for an increased risk of pre-eclampsia. In particular the abnormal level is either a decreased level of transthyretin or an increased level of alpha-1 B-glycoprotein or antithrombin III .

The invention also relates to a method for ameliorating the effects of a preterm delivery for the newborn, comprising the steps of identifying women with an increased risk of pre-eclampsia according to the invention and treating women with an increased risk of pre-eclampsia with a steroid or another effective medication in order to promote foetal lung maturation.

The invention also relates to a method for screening or pre-screening pregnant women to identify those pregnant women with a low risk of developing hypertensive disorders, the method comprising the steps of obtaining a biological sample from a pregnant patient after week 12 and prior to week 37 of pregnancy, in the biological sample, determining the level of at least one biomarker selected from the group consisting of alpha-1 B-glycoprotein, antithrombin III and transthyretin, comparing the level of the at least one biomarker with a level characteristic of normal pregnancies wherein a normal level of said at least one biomarker is indicative for a pregnancy with a low risk of developing hypertensive disorders.

### Legend to the figure

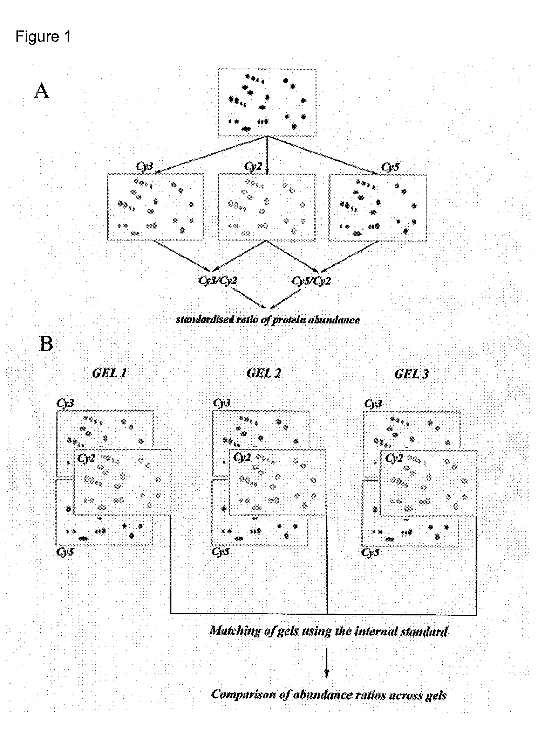

Figure 1. Statistical Analysis of 2D-DIGE Gels. (A) Following imaging, protein spots labelled with either Cy3 or Cy5 are standardised against the pooled internal control spot labelled with Cy2. Gel to gel variation is removed as the samples have all been resolved in the same gel. This is performed in the DIA module of DeCyderTM 2-D Differential Analysis Software. (B) The internal standard is also used for matching and comparing protein spot abundance across gels (performed by the DeCyderTM BVA module). This enables the statistical comparison of protein abundance changes in multiple samples.

### Detailed description of the invention

The present invention relates to a screening assay which provides an early, biochemical indication of increased risk of pre-eclampsia. The method can provide an indication of impending pre-eclampsia as early as three to four months prior to delivery, such as 2 or 1 month or 4, 3, 2 or 1 week. The method allows early intervention in the course of pre-eclampsia and provides an additional factor which can indicate those pregnancies at greatest risk.

The method comprises obtaining a biological sample from the pregnant patient after about week 12 of pregnancy and prior to about week 36 or 37 and determining the level of one or more biomarkers as disclosed herein in the sample. The presence of an abnormal level of the biomarker in the sample indicates a patient who is at risk for pre-eclampsia.

In particular, the present invention provides a method for identifying women with an increased risk of pre-eclampsia comprising the steps of obtaining a biological sample from a pregnant patient after week 12 and prior to week 37 of pregnancy and in the biological sample, determining the level of at least one biomarker selected from the group consisting of alpha-1B-glycoprotein, antithrombin III and transthyretin and comparing the level of the at least one biomarker with a level characteristic of pregnancies that proceed to term, wherein an abnormal level of said at least one biomarker is indicative for an increased risk of pre-eclampsia.

In contrast to the prior art methods, the method according to the invention is capable of predicting pre-eclampsia months in advance, whereas the prior art provides assays that may be used when clinical symptoms of pre-eclampsia are already apparent.

As used herein, the term "pre-eclampsia" is defined according to criteria established by the committee on Terminology of the American College of Obstetrics and Gynecology, such as a blood pressure of at least 140/90 mm Hg and urinary excretion of at least 0.3 grams of protein in a 24-hour urinary protein excretion (or at least +1 or greater on dipstick testing), each on two occasions 4-6 hours apart. The term pre-eclampsia is used so as to include severe pre-eclampsia.

As used herein, "severe pre-eclampsia" is also defined in accordance with established criteria, as a blood pressure of at least 160/110 mm Hg on at least 2 occasions 6 hours apart and greater than 5 grams of protein in a 24- hour urinary protein excretion or persistent +3 proteinuria on dipstick testing. Severe preeclampsia may include HELLP syndrome (hemolysis, elevated liver enzymes, low platelet count). Other elements of severe preeclampsia may include in-utero growth restriction (IUGR) in less than the 10 % percentile according to the US demographics, persistent neurologic symptoms (headache, visual disturbances), epigastric pain, oliguria (less than 500 mL/24 h), serum creatinine greater than 1.0 mg/dL, elevated liver enzymes (greater than two times normal), thrombocytopenia (< 100,000 cells/[mu]L).

The term "abnormal level" is meant to indicate either a decreased level of transthyretin or an increased level of alpha-1 B-glycoprotein or antithrombin III.

The term normal pregnancy is meant to indicate a pregnancy that proceeds to term without the complication of pre-eclampsia. Preferably, a normal pregnancy is a pregnancy without any other complications, such as in particular preterm delivery or a growth retardation of the foetus.

A biological sample may actually be any sample obtained form a pregnant women, the results presented in the present specification are based on sera obtained from pregnant woman. One may also conceive to utilize other biological fluids such as amniotic fluid, vaginal excretions, saliva and urine. Preferably, however, the biological sample is a blood, plasma or serum sample. It is a great advantage when a screening assay can be performed on a blood, serum or plasma sample, since these samples can be taken from a patient with minimal discomfort.

The levels of a particular biomarker provided herein are to be compared with the levels of that same biomarker in the same type of sample taken from a patient or a number of patients that experienced term delivery. Preferably those control samples are from a normal pregnancy of the same gestational period or age as the period or age of the patient screened for the risk of pre-eclampsia

For an even higher precision of the method, the samples may be age matched.

It will be understood that the level of markers as disclosed herein can be measured directly or indirectly by a variety of different techniques. However, if the method is to be used as a screening assay, an immunological method is preferred. Antibodies against all the biomarkers provided herein are readily available and a person skilled in the art will know how to design and develop an immunological assay based on such antibodies. For some of the markers, even commercial assays are available.

Particularly preferred immunological methods are radioimmunoassays and enzyme-linked immunosorbent assays (ELISAs) because they are particularly amenable to routine laboratory practice.

A significant problem for premature babies is lung immaturity. Foetal lungs can be matured by the administration of beta methasone. However, if premature delivery is indicated, the beta methasone needs to be administered quickly in order to permit lung maturation to occur prior to delivery.

When (severe) pre-eclampsia is diagnosed according to a method as described above, immediate delivery may be indicated. The chances for survival of the foetus increase when at that point in time effective medication is supplied to promote foetal lung maturation. The invention is therefore also directed to a method for ameliorating the effects of a preterm delivery for the newborn, comprising the steps of identifying women with an increased risk of pre-eclampsia according to the invention and treating women with an increased risk of pre-eclampsia with a steroid or another effective medication in order to promote foetal lung maturation.

Since the methods according to the invention also have a good negative predictive value, the invention is also directed towards a method for screening or pre-screening pregnant women to identify those pregnant women with a low risk of developing hypertensive disorders, the method comprising the steps of obtaining a biological sample from a pregnant patient after week 12 and prior to week 37 of pregnancy, in the biological sample, determining the level of at least one biomarker selected from the group consisting of alpha-1 B-glycoprotein, antithrombin III and transthyretin, comparing the level of the at least one biomarker with a level characteristic of pregnancies that proceed to term, wherein a normal level of said at least one biomarker is indicative for a pregnancy with a low risk of developing hypertensive disorders.

### Patients to be tested

The present method can be used on any pregnant woman following about 12 weeks gestation and prior to term pregnancies (week 36 or 37). In addition to screening any pregnant woman to determine whether the occurrence of pre-eclampsia is likely, the patients who are preferably screened are those patients with clinically intact membranes in a high risk category for pre-eclampsia.

Any pregnant woman at 12 or more weeks gestation with clinically intact membranes and having one or more risk factors for pre-eclampsia is preferably tested throughout the risk period; i.e., until about week 34 to 37.

Preferably, the method is used on samples taken in the second trimester of pregnancy. The levels of the biomarkers disclosed in this application may be measured in blood, preferably in serum or plasma.

Preferably, the samples should be tested immediately after preparation. Storage up to several days under appropriate conditions was shown not to affect the results, nevertheless, addition of protein stabilisers and protease inhibitors such as kallikrein inhibitors (PMSF) is recommended.

### Assay procedures

Abnormal levels of alpha-1B glycoprotein were found to be indicative of the diagnosis of pre-eclampsia. On average, patients with pre-eclampsia exhibited a level of alpha-1B glycoprotein that was about 1.35 times higher as compared to patients with a normal pregnancy.

Levels of alpha-1B glycoprotein were measured using two dimensional differential gel electrophoresis (2D-DIGE) as described in the examples. For the routine determination of alpha-1B glycoprotein in the clinical laboratories, an immunological method is preferred. Such methods are described in example 10.

The gene encoding alpha-1B glycoprotein has been described in Strausberg R.L. et al Proc. Natl. Acad. Sci. U.S.A. 99:16899-16903(2002), thus allowing the production of specific antibodies. Moreover, rabbit antibodies against human alpha-1B glycoprotein are commercially available from Genway Biotech Inc in San Diego and from Aviva Systems Biology San Diego.

Abnormal levels of antithrombin III were also found to be indicative of the diagnosis of pre-eclampsia. On average, patients with pre-eclampsia exhibited a level of antithrombin III that was about 2 times higher as compared to patients with a normal pregnancy.

Levels of antithrombin III were measured using two dimensional differential gel electrophoresis (2D-DIGE) as described in the examples. For the routine determination of antithrombin III in the clinical laboratories, an immunological method is preferred. Such methods are described in example 10.

Mouse monoclonal antibodies against human antithrombin III are commercially available from AntibodyShop A/S in Gentofte, Denmark.

Abnormal levels of transthyretin were also found to be indicative of the diagnosis of pre-eclampsia. On average, patients with pre-eclampsia exhibited a level of transthyretin that was about 1.6 times lower as compared to patients with a normal pregnancy.

Levels of transthyretin were measured using two dimensional differential gel electrophoresis (2D-DIGE) as described in the examples. For the routine determination of transthyretin in the clinical laboratories, an immunological method is preferred. Such methods are described in example 10.

The gene encoding transthyretin has been described by Mita et al., in Biochem. Biophys. Res. Commun. 124:558-564(1984), thus allowing the production of specific antibodies. Moreover, chicken antibodies against human transthyretin are commercially available from Genway Biotech Inc in San Diego and from Aviva Systems Biology San Diego.

An ELISA for the sensitive determination of transthyretin has been described by Vatassery et al., in Clin Chim. Acta, 1991, 197; 19-25.

### Interpretation of results

Current biomarkers for the determination of the risk of pre-eclampsia suffer from the drawback of a low sensitivity. The markers identified herein were found to be remarkably sensitive, at an excellent specificity.

When the lowest measured value of the normal (term delivery, non hypertensive disorder) samples was taken as the cut-off value, the method employing anti-thrombin III was found to be 100% specific and 100% sensitive (table 3). Also, when the highest measured value of the normal (term delivery, non hypertensive disorder) samples was taken as the cut-off value, the method employing transthyretin was found to be 100% specific and 100% sensitive (table 3).

The methods employing markers alpha-1B-glycoprotein and antithrombin III were found to be very robust; even when the mean value of the normal samples plus two times the standard deviation was taken as the cut off value, an excellent sensitivity was obtained of 75 and 100% respectively while the specificity remained at 100% (table 2).

The values obtained for transthyretin varied remarkably within the normal (term delivery without hypertension) group as well as within the pre-eclampsia group (tables 1 and 2). At an average standardised abundance of 2,23, the standard deviation of 6 samples was 0.44. Due to this high variability, the negative predictive value of this biomarker was somewhat lower than the other two markers from the group of 3 markers disclosed herein. Nevertheless, transthyretin is considered a valuable and specific marker for pre-eclampsia since the average standardised abundance of the normal delivery group is about 1.6 times higher than the value of the pre-eclampsia group. The sensitivity of this marker was 100% at a specificity of 100% when an appropriate cut-off value was chosen.

Antithrombin III was found to be the most robust marker. It exhibited a specificity and sensitivity of 100%, even if the cut-off value was chosen at average normal value plus 2 times the standard deviation (Table 2).

An even more sensitive method could be obtained when several of the markers presented herein were combined, i.e. when combination assays were used. This is completely in line with the aetiology of pre-eclampsia as it is usually considered to have multiple causes.

Any combination of two markers chosen from the three markers presented herein, resulted in a method with an increased sensitivity. Hence, the invention relates to a method as described above, wherein at least two biomarkers were used, selected from the group consisting of alpha-1B-glycoprotein, antithrombin III and transthyretin.

Sensitivity of the method could even be further improved when all three biomarkers were used in a method according to the invention. Even when the cut-off value of the assays was chosen at the average normal value plus or minus two times the standard deviation of the normal population, a combination assay could be created that was 100% specific and 100% specific. Even more importantly, it was found that on average at least two markers were positive for each of the four samples obtained from the pre-eclampsia patients (table 2).

A particularly useful method was obtained when markers alpha-1B-glycoprotein and antithrombin III were combined. Even if the cut-off value was chosen at average normal value plus 3 times the standard deviation, this method yielded a sensitivity and specificity of 75% and 100% respectively.

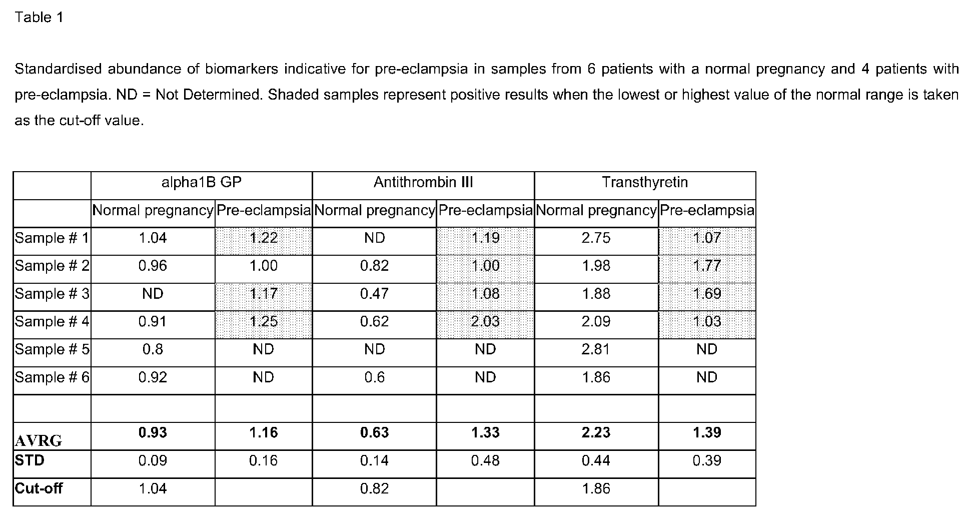

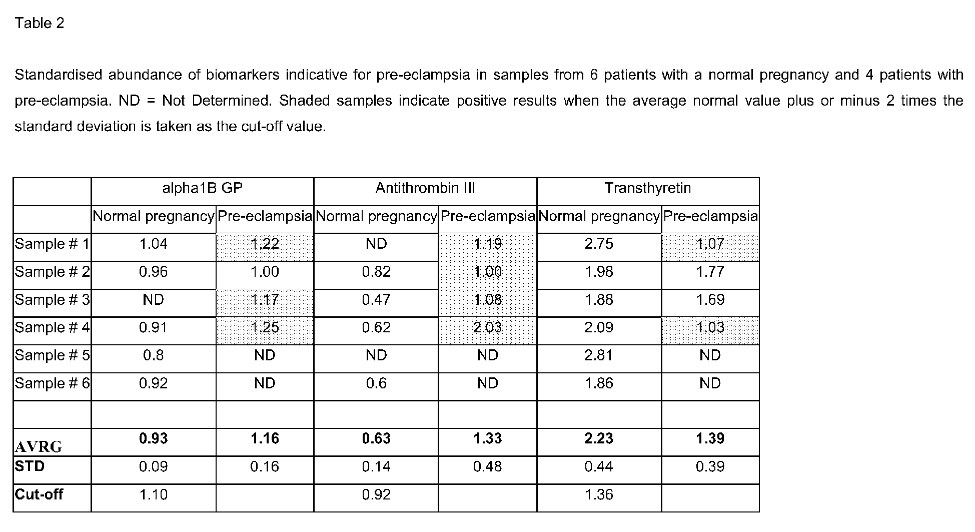

**Table 3.**

| | | | | | | | | |
|---|---|---|---|---|---|---|---|---|
| Sensitivity, specificity, positive predictive value and negative predictive value of biomarkers for pre-eclampsia. Cut-off value = lowest/highest normal value. | | | | | | | | |

| Biomarker | False positive | False negative | True positive | True negative | Specificity [%] | Sensitivity [%] | PPV [%] | NPV [%] |
|---|---|---|---|---|---|---|---|---|
| Alpha-1B-glycoprotein | 0 | 1 | 3 | 5 | 100 | 75 | 100 | 83 |
| Antithrombin III | 0 | 0 | 4 | 4 | 100 | 100 | 100 | 100 |
| Transthyretin | 0 | 0 | 4 | 6 | 100 | 100 | 100 | 100 |

### Examples

### Example 1: Plasma samples

Samples were obtained prospectively from pregnant women who were followed until delivery and clinical conditions identified and monitored. Within a cohort of 500 women who experienced pre-eclampsia period were identified. A group of normal pregnant women were also identified. Blood plasma samples from these two groups taken at 24 weeks of pregnancy were subjected to two dimensional difference gel electrophoresis (2D-DIGE) analysis. Using 2D-DIGE, the plasma protein profiles of 6 normal pregnant women were compared with plasma proteins profiles from 6 women who experienced pre-eclampsia.

### Example 2: Depletion of HSA and IgG and Preparation of Blood Proteins

Human Serum Albumin (HSA) and gamma-immunoglobulin (IgG) account for up to 60% of total blood protein content. This is problematic for protein-based studies of blood because these proteins can mask and prevent the analysis of lower abundance proteins. Samples were depleted of HSA and IgG using the Albumin and IgG Removal Kit (GE Healthcare, Piscataway, NJ, USA) according to the manufacturer's protocol. In brief, extracted samples were incubated with an anti-HSA/anti-IgG resin for 30mins at room temperature. Unbound proteins were separated from the absorption matrix using a mini-spin cartridge and centrifuged at 1000rpm for 2mins. To concentrate protein and remove excess salts, the HSA/IgG depleted samples were precipitated by adding 4 volumes of ice-cold acetone to each sample. The solution was then incubated for 3 hrs at -20°C and then centrifuged at 13000rpm for 15mins at 4°C. Samples were resuspended in 50µl of extraction buffer (7M urea, 2M thiourea, 30mM Tris, 4% w/v CHAPS, pH 8.5). Protein concentration estimation was performed using the 2-D Quant Kit (GE Healthcare).

### Example 3: Fluorescent Cy-dye labelling of samples

Prior to labelling, cyanine dyes Cy2, 3 and 5 (GE Healthcare) were reconstituted in anhydrous DMF. 50µg protein from normal spontaneous labour and the pre-eclampsia group were labelled with 400pM of either Cy3 or Cy5 dye on ice for 30mins in the dark. Half of each sample population was labelled with Cy3 and the other with Cy5 to account for any dye binding bias. An internal pooled sample was created by mixing 25µg of each sample and labelling as above with Cy2. Dye labelling reactions were stopped by adding 10mM lysine and incubating for 10mins on ice in the dark. Cy3 and Cy5-labelled samples were mixed with the Cy2-labelled pooled control and made up to a volume of 450µl with rehydration buffer (7M urea, 2M thiourea, 4% w/v CHAPS, 2mg/mL DTT and 1% v/v pH3-1 0 immobilised pH gradient (IPG) buffer).

### Example 4: Two-dimensional gel electrophoresis

In-gel rehydration of IPG strips (non linear, pH3-10, 24cm; GE Healthcare) was performed with the Cy-labelled samples overnight for 12hrs at room temperature under mineral oil (Sigma, St. Louis, MO, USA). Isoelectric focusing was performed using a Multiphor II unit (GE Healthcare) for a total of 58800Vhrs using the following parameters; hold at 300V for 900Vhrs; ramp and hold at 1000V for 3900Vhrs; ramp and hold to 8000V for 13000Vhrs; hold at 8000V for 41000Vhrs. After focusing strips were equilibrated and reduced in equilibration buffer (30% v/v glycerol, 2% w/v SDS, 7M urea, trace bromophenol blue) with 0.5% DTT for 15mins at room temperature and then in equilibration buffer containing 4.5% iodacetamide for 15mins at room temperature. Equilibrated strips were applied on homogeneous 10% acrylamide gels (25.5 x 20.5cm) cast in low-fluorescence plates using the Ettan™ DALT *six* Electrophoresis Casting System (GE Healthcare). This enabled the production of six simultaneously-cast gels using the same batch of acrylamide gel stock solution for each gel. Second-dimensional electrophoretic separation was carried out at 2.5W/gel constant power for 30mins then 100W (total) constant power using a peltier- cooled Ettan DALT II electrophoresis unit (GE Healthcare) until the bromophenol dye front reached the bottom of the gel.

### Example 5: Imaging and Analysis of 2D-DIGE gels

Fluorescence image acquisition was performed using the Typhoon 9400 Variable Mode Imager (GE Healthcare). Each gel was scanned at the following excitation/emission wavelengths; 480/530nm (Cy2), 520/590nm (Cy3) and 620/680nm (Cy5). Gel analysis was conducted using the DeCyder V5.0 Biological Variation software module (GE Healthcare). This enabled spot matching between gels and also to quantify and standardise protein spot data. Spot matching was validated by manual inspection of gel spots. Statistically significant protein abundance changes were identified via Student's T-test. Proteins that were present in either all gels or at least in the spontaneous normal labour group with a p-value of p<0.05 or less were chosen as proteins for identification.

### Example 6: Utilisation of the internal standard

The primary advantage of 2D-DIGE over other proteomic techniques is the inclusion of an internal standard control. This allows compensation for variation within gels and across gels in an experimental series. The internal control was created by pooling equal quantities of all samples to be assessed in each experiment. The pool was labelled with one of the Cy fluorophores (Cy2) and applied to every gel in the experiment containing Cy3 and Cy5 labelled samples. This means that every individual protein spot on the gel was be represented by the Cy2-labelled internal control. Therefore each protein spot could be standardised to its own control spot as well as across all gels in the experiment (Figure 1). In this way gel-to-gel variation was eliminated. This also allowed for the discrimination between system variation and true biological variation.

### Example 7: Protein sequencing

Two sequencing gels were prepared by performing 2D-PAGE on 500µg and 1000µg of pooled myometrium proteins. The gels were fixed in 50% methanol, 7% acetic acid for 30mins at room temperature and then stained with SYPRO Ruby (Invitrogen, California, USA) overnight also at room temperature. The gels were then washed in 10% methanol, 7% acetic acid and briefly rinsed in distilled water before being visualised using a UV illuminator. Proteins of interest were excised from the gels and destained in 50mM ammonium bicarbonate in 50% v/v methanol for 3 washes of 40mins each. Gel plugs were then dried at 37°C for 2hrs and then incubated in 40ng/µl trypsin (Promega, Madison, WI, USA) in 20mM ammonium bicarbonate at 37°C for 3 hours. Protein sequencing was performed via matrix-assisted laser desorption time-of-flight mass spectrometry (MALDI-ToF) using the Ettan MALDI-ToF Pro (GE Healthcare). Approximately 1 µl tryptic peptide was mixed with 1µl a-cyano-4-hydroxycinnamic acid matrix (5 mg/ml in 50% acetonitrile, 0.1% trifluroacetic acid) and 1µl of this mixture was spotted in duplicate onto the Maldi target tray. Peptide mass fingerprinting was performed alongside peptide standards (company) in reflectron mode. The external standard was use to calibrate the mass spectrometer prior to acquisition of sample MS data acquisition. Sequence data was used to interrogate the Swiss-Prot and NCBInr databases. As a further control measure, the isoelectric point and molecular weight of positively identified proteins were checked against the region of the pick gel they were excised from.

The following procedures are constructively reduced to practice and therefore described in the present tense, as opposed to the previous examples which were carried out in the laboratory and which were therefore set forth in the past tense.

### Example 8: Purification of polypeptides useful in the Invention

The polypeptide markers that are useful in the invention can be produced by first cloning the corresponding polynucleotides into a mammalian expression vector using established protocols that are familiar to those in skill of the art. In brief, polynucleotides of the invention are amplified and isolated via agarose gel electrophoresis and gel excision. The polynucleotides are subsequently cloned into appropriate vectors to facilitate in-frame cloning. Proteins may for instance be expressed with a polyhistidine metal-binding tag. The vectors with polynucleotides of the invention inserted can then be transfected into a mammalian cell line like COS-7 for high-level expression of the corresponding polypeptides.

The primary nucleotide sequence of all markers useful in the invention may be obtained from Genbank (table 4).

The polypeptides useful in the invention that are produced by the transfected mammalian cell line can then be purified according to established protocols that are familiar to those of skill in the art, for example using the Ni-NTA Magnetic Agarose Beads protein purification protocol, Qiagen, Valencia, Calif. In brief, assays utilizing Ni-NTA Magnetic Beads involve capture of the His-tagged protein-from a cell lysate or a purified-protein solution-followed by washing, binding of interaction partners, further washing, and finally elution of the interacting partner from the still immobilized His-tagged protein or elution of the interacting-partner-His-tagged-protein complex. Between each step, the beads are collected by attracting them to the side of the vessel, after placing near a magnet for 30-60 seconds. Purification procedures may even use crude cell extracts for binding of His-tagged protein.

The resulting purified polypeptides of the invention are then quantified, lyophilized and stored at 4 degrees Celcius or below, and may be used for standards in diagnostic kits or for the generation of antibodies.

### Example 9: Production of Specific Antibodies for the Polypeptides of the Invention

Polyclonal antibodies are produced by DNA vaccination or by injection of peptide antigens into rabbits or other hosts. An animal, such as a rabbit, is immunized with a peptide advantageously conjugated to a carrier protein, such as BSA (bovine serum albumin) or KLH (keyhole limpet hemocyanin). The rabbit is initially immunized with conjugated peptide in complete Freund's adjuvant, followed by a booster shot every two weeks with injections of conjugated peptide in incomplete Freund's adjuvant. Antibodies specific for a polypeptide of the invention are affinity purified from rabbit serum using peptides corresponding to the polypeptides of the invention, for instance coupled to Affi-Gel 10 (Bio-Rad), and stored in phosphate- buffered saline with 0.1 % sodium azide. To determine that the polyclonal antibodies are specific for a polypeptide of the invention, an expression vector encoding a polypeptide of the invention is introduced into mammalian cells. Western blot analysis of protein extracts of non-transfected cells and the cells containing a polypeptide of the invention is performed using the polyclonal antibody sample as the primary antibody and a horseradish peroxidase-labeled anti-rabbit antibody as the secondary antibody. Detection of a band in the cells containing a polypeptide of the invention and lack thereof in the control cells indicates that the polyclonal antibodies are specific for the polypeptide of the invention in question.

Monoclonal antibodies are produced by injecting mice with a peptide derived from a marker polypeptide of the invention, with or without adjuvant. Subsequently, the mouse is boosted every 2 weeks until an appropriate immune response has been identified (typically 1-6 months), at which point the spleen is removed. The spleen is minced to release splenocytes, which are fused (in the presence of polyethylene glycol) with murine myeloma cells. The resulting cells (hybridomas) are grown in culture and selected for antibody production by clonal selection. The antibodies are secreted into the culture supernatant, facilitating the screening process, such as screening by an enzyme-linked immunosorbent assay (ELISA). Alternatively, humanized monoclonal antibodies are produced either by engineering a chimeric murine/human monoclonal antibody in which the murine- specific antibody regions are replaced by the human counterparts and produced in mammalian cells, or by using transgenic "knock out" mice in which the native antibody genes have been replaced by human antibody genes and immunizing the transgenic mice as described above.

Suitable antibodies may also be commercially obtained as described above.

### Example 10: Diagnostic Kit for markers according to the Invention

A diagnostic kit can be made in order to determine the levels of selected polypeptides of the invention that are present in samples obtained from a patient who is undergoing pregnancy. Such samples may conveniently be from blood, blood plasma, blood serum, amniotic fluid, saliva or urine.

A diagnostic kit may comprise some or all of the following components: 1) one or more standards comprised of one or more polypeptides of the invention, prepared as described in example 8; 2) an antibody or a plurality of antibodies that are specific for the polypeptides of the invention that are being tested by the kit, prepared as described in example 9; 3) written instructions; 4) diluents for samples and the standards; 5) a wash buffer; 6) color reagents; 7) stop solution; and 8) an antibody carrier such as polystyrene beads or a lateral flow device or a microplate with bound antibody.

An example of such a kit is a quantificative ELISA (enzyme-linked immunosorbent assay) that determines the concentration or concentrations of a polypeptide or polypeptides according to the invention. The principle of the assay is to use the quantitative sandwich enzyme immunoassay technique where a monoclonal or polyclonal antibody selective for binding a polypeptide of the invention is pre-coated onto a carrier such as a microplate into its wells. The standards and sample are then pipetted into the wells and any of the polypeptide of the invention that is present is bound to this immobilized antibody. Next, the wells are washed with washing buffer, and an enzyme-linked monoclonal or polyclonal antibody that is specific for the polypeptide of the invention is added to the wells. Washing again is performed, then a substrate solution is added to the wells. Color subsequently develops in proportion to the amount of polypeptide of the invention that is bound in the first step. The color development is stopped using a stop solution, and the intensity of the color is measured by a microplate reader.

Alternatively, a lateral flow assay may be developed. Such lateral flow assays have the potential to be a cost-effective, fast, simple, and sensitive method, for instance for on-site screening assays. The principle is well known to the skilled person and consist essentially of a carrier that allows a lateral flow to occur wherein either the sample or the detection reagent is displaced form one location on the carrier to another. There are many formats of lateral flow assays suitable for the method according to the invention, the skilled person will know how to choose and optimize a particular format.

### Example 11: Diagnostic Chip for Polypeptides of the Invention

The levels of polypeptides of the invention in biological samples can also be determined through the use of ProteinChip technology (Wright Jr., et al. Expert Review of Molecular Diagnositcs 2002, Vol. 2 pp. 549-63 hereby incorporated herein by reference). This process includes the additon of a few microliters of sample onto the ProteinChip surface, which has both chemical (e.g., anionic, catioinic, hydrophobic, hydrophilic, metal) and biochemical (antibody, receptor, DNA, enzyme probes attached to its surface. Subsequently, binding occurs between the polypeptides of the invention and their respective probes, and the chip is washed at least three times in order to elute unbound protein, lipids, salts, and other substances. Next, an energy-absorbent molecule (EAM) such as sinapinic acid is added to the chip, and the chip is placed into a ProteinChip Reader which measures the molecular mass of the bound polypeptides. Peaks are subsequently produced from the ProteinChip Reader that are used to quantify the amount of polypeptides of the invention in the sample.

**Table 4: List of high molecular weight proteins identified as biomarkers of pre-eclampsia.**

| **Protein ID** | **Swiss Prot accession # and entry name** | **Synonyms** | **MW of unprocessed precurser (KDa)** | **pl** | **Peptides Sequenced** | **% sequence coverage** |
|---|---|---|---|---|---|---|
| Alpha-1B-glycoprotein | P04217 AIBG_HUMAN | | 54.273 | 5.6 | 5 | 12.3 |
| Antithrombin III | P01 008 ANT3_HUMAN | ATIII | 52.602 | 6.3 | 6 | 11.9 |
| Transthyretin | Q549C7 Q549C7_HUMAN | Prealbumin, TBPA, TTR, ATTR | 15.887 | 5.3 | 6 | 74.4 |

## Claims

1. A method for identifying women with an increased risk of pre-eclampsia comprising the steps of
a) Obtaining a biological sample from a pregnant patient after week 12 and prior to week 37 of pregnancy
b) In the biological sample, determining the level of at least one biomarker selected from the group consisting of alpha-1B-glycoprotein, antithrombin III and transthyretin,
c) Comparing the level of the at least one biomarker with a level characteristic of normal pregnancies,
wherein an abnormal level of said at least one biomarker is indicative for an increased risk of pre-eclampsia.

2. A method according to claim 1 wherein the abnormal level is either a decreased level of transthyretin or an increased level of alpha-1 B-glycoprotein or antithrombin III.

3. A method according to claim 1 or 2 which is an immunological method.

4. A method according to claim 3 which is a radioimmunoassay or an ELISA or a lateral flow assay.

5. A method according to claims 1 - 4 wherein the at least one biomarker is selected from the group consisting of alpha-1 B-glycoprotein and antithrombin III.

6. A method according to claims 1 - 5 wherein the biological sample is blood or plasma or serum.

7. A method for ameliorating the effects of a preterm delivery for the newborn, comprising the steps of identifying women with an increased risk of pre-eclampsia according to claims 1 - 6 and treating women with an increased risk of preeclampsia with a steroid or another effective medication in order to promote foetal lung maturation.

8. A method for screening or pre-screening pregnant women to identify those pregnant women with a low risk of developing hypertensive disorders, the method comprising the steps of
a) Obtaining a biological sample from a pregnant patient after week 12 and prior to week 37 of pregnancy
b) In the biological sample, determining the level of at least one biomarker selected from the group consisting of alpha-1B-glycoprotein, antithrombin III and transthyretin,
c) Comparing the level of the at least one biomarker with a level characteristic of a normal pregnancy,
wherein a normal level of said at least one biomarker is indicative for a pregnancy with a low risk of developing hypertensive disorders.
